# EUROPEAN PATENT APPLICATION

(11) **EP 4 495 242 A1**
(43) Date of publication of application: **22.01.2025**
(21) Application number: 23770873.0
(22) Date of filing: 16.03.2023
(51) Int. Cl.: C12N 15/113, A61K 31/712, A61K 31/7125, A61K 48/00, A61P 25/00, A61P 43/00

(54) **ANTISENSE NUCLEIC ACID CAPABLE OF INHIBITING BIOSYNTHESIS OF CHONDROITIN SULFATE**

(30) Priority: 17.03.2022 JP 2022042954
(71) Applicant: Aichi Medical University, Aichi 480-1195 (JP); Luxna Biotech Co., Ltd., Suita-shi, Osaka 565-0871 (JP)
(72) Inventor: TAKEUCHI Kosei, Nagakute-shi, Aichi 480-1195 (JP); SASAKURA Hiroyuki, Nagakute-shi, Aichi 480-1195 (JP); SUZUKI Takao, Suita-shi, Osaka 565-0871 (JP); YAMAGAMI Masaki, Suita-shi, Osaka 565-0871 (JP); KAWANOBE Takaaki, Suita-shi, Osaka 565-0871 (JP); SHRESTHA Ajaya Ram, Suita-shi, Osaka 565-0871 (JP); MIYASAKA Yui, Suita-shi, Osaka 565-0871 (JP); UMEMOTO Tadashi, Suita-shi, Osaka 565-0871 (JP); SEKIGUCHI Kazuo, Suita-shi, Osaka 565-0871 (JP); KOBORI Takuro, Suita-shi, Osaka 565-0871 (JP)
(74) Representative: Mewburn Ellis LLP
(86) International application number: PCT/JP2023/010273
(87) International publication number: WO 2023/176920

(57) **Abstract**

This invention provides a highly functional antisense oligonucleotide that inhibits biosynthesis of chondroitin sulfate. The invention also provides an antisense oligonucleotide that suppresses expression of the chondroitin sulfate N-acetylgalactosaminyl-transferase-1 (CSGalNAc-T1) gene and has the properties (i) to (iv): (i) an antisense oligonucleotide is 13-to 25-mer in length; (ii) an antisense oligonucleotide comprises a gap region, a 3'-wing region bound to the 3' end of the gap region, and a 5'-wing region bound to the 5' end of the gap region; (iii) nucleotides in the 3'-wing region and in the 5'-wing region include at least one bridged nucleic acid and the bridged nucleic acid is scpBNA or AmNA; and (iv) an antisense oligonucleotide comprises a nucleotide sequence selected from the group consisting of SEQ ID NOs: 2 to 31 or a sequence derived therefrom by substitution, deletion, or insertion of 1 or 2 nucleic acid bases.

## Description

### Technical Field

The present invention relates to an antisense oligonucleotide that inhibits biosynthesis of chondroitin sulfate.

### Background Art

A spinal cord injury (SCI) is a pathological state in which the spinal cord is injured typically by application of strong pressure to the spinal column. Once injured, the central nervous system including the spinal cord would never be restored and regenerated, and therefore patients having a spinal cord injury often suffer from severe motor function disorders.

Chondroitin sulfate (CS) is a type of glycosaminoglycan that is extensively distributed in extracellular matrices of animals. Chondroitin sulfate has a structure in which sulfuric acid is bound to a sugar chain in which two sugars: D-glucuronic acid (GlcA) and N-acetyl-D-galactosamine (GalNAc), are repeated. It is known that, after injury of the spinal cord, chondroitin sulfate is produced in the spinal cord, and the produced chondroitin sulfate serves as a strong inhibitor of axon regeneration.

Chondroitin sulfate N-acetylgalactosaminyl-transferase-1 (CSGalNAcT1) and chondroitin sulfate N-acetylgalactosaminyl-transferase-2 (CSGalNAcT2) are enzymes that are involved in biosynthesis of chondroitin sulfate.

The present inventors have reported that lowered synthesis of chondroitin sulfate after the spinal cord injury and promoted recovery from the spinal cord injury would be observed in CSGalNAcT1 knockout mice (Non Patent Literature 1 and Non Patent Literature 2). The present inventors have also reported an antisense oligonucleotide that can inhibit biosynthesis of chondroitin sulfate by inhibiting GSGalNAcT1 and/or GSGalNAcT2 (Patent Literature 1).

### Citation List

### Patent Literature

Patent Literature 1: WO 2018/180005

### Non Patent Literature

Non Patent Literature 1: Watanabe, T., et al., Biochem. J., 2010, 432: 47-55
Non Patent Literature 2: Takeuchi, K., et al., Nature Communication, 2013, 4: 2740

### Summary of Invention

### Technical Problem

The objective of the present invention is to provide a highly functional antisense oligonucleotide that inhibits biosynthesis of chondroitin sulfate.

### Solution to Problem

The present inventors have conducted concentrated studies in order to attain the above objective. As a result, an antisense oligonucleotide with high functionality has been discovered that would inhibit the CSGalNAcT1 gene and inhibit biosynthesis of chondroitin sulfate. This has led to the completion of the present invention.

Specifically, the present invention includes the following,
(1) An antisense oligonucleotide that suppresses expression of the chondroitin sulfate N-acetylgalactosaminyl-transferase-1 (CSGalNAcT1) gene and has the following characteristics:
   (i) an antisense oligonucleotide is 13- to 25-mer in length;
   (ii) an antisense oligonucleotide comprises a gap region, a 3'-wing region bound to the 3' end of the gap region, and a 5'-wing region bound to the 5' end of the gap region;
   (iii) nucleotides in the 3'-wing region and in the 5'-wing region include at least one bridged nucleic acid and the bridged nucleic acid is scpBNA or AmNA; and
   (iv) an antisense oligonucleotide comprises a nucleotide sequence selected from the group consisting of SEQ ID NOs: 2 to 31 or a sequence derived therefrom by substitution, deletion, or insertion of 1 or 2 nucleic acid bases.
(2) The antisense oligonucleotide according to (1), wherein the nucleotides in the 3'-wing region and in the 5'-wing region include at least one scpBNA.
(3) The antisense oligonucleotide according to (1) or (2), wherein at least one internucleotide bond is a phosphorothioate bond.
(4) The antisense oligonucleotide according to any of (1) to (3), wherein at least one internucleotide bond in the 3'-wing region and in the 5'-wing region is a phosphodiester bond.
(5) The antisense oligonucleotide according to (4), wherein the proportion of phosphodiester (PO) bonds relative to the total of phosphorothioate (PS) bonds and PO bonds (PO/(PS+PO)) of the internucleotide bonds in the 3'-wing region and in the 5'-wing region is less than 0.5.
(6) The antisense oligonucleotide according to any of (1) to (5), wherein the gap region is 7- to 17-mer in length, the 3'-wing region is 2- to 6-mer in length, and the 5'-wing region is 2-to 6-mer in length.
(7) The antisense oligonucleotide according to any of (1) to (6), which has a nucleotide sequence selected from the group consisting of SEQ ID NOs: 24, 26, 27, 29, and 31 or a nucleotide sequence derived therefrom by substitution, deletion, or insertion of 1 or 2 nucleic acid bases.
(8) A pharmaceutical composition comprising the antisense oligonucleotide according to any of (1) to (7) used for treating a disease or condition related to an increase in chondroitin sulfate.
(9) The pharmaceutical composition according to (8), wherein the disease or condition related to an increase in chondroitin sulfate is a spinal cord injury.
(10) The pharmaceutical composition according to (9), which is administered at the acute phase or the subacute phase of a spinal cord injury.
(11) The pharmaceutical composition according to (10), which is administered at the subacute phase of a spinal cord injury.

The description incorporates the contents disclosed by JP Patent Application No. 2022-042954, based on which the priority of the present application claims.

### Advantageous Effects of Invention

The present invention provides a highly functional antisense oligonucleotide that inhibits biosynthesis of chondroitin sulfate.

### Brief Description of Drawings

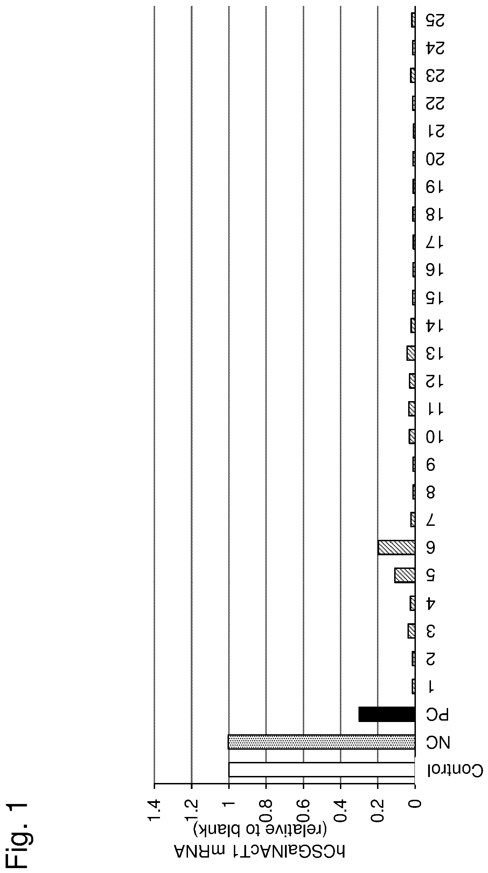

[Figure 1] Figure 1 shows a graph illustrating effects of the antisense oligonucleotide for suppressing expression of the CSGalNAcT1 gene in the human U251 cells.

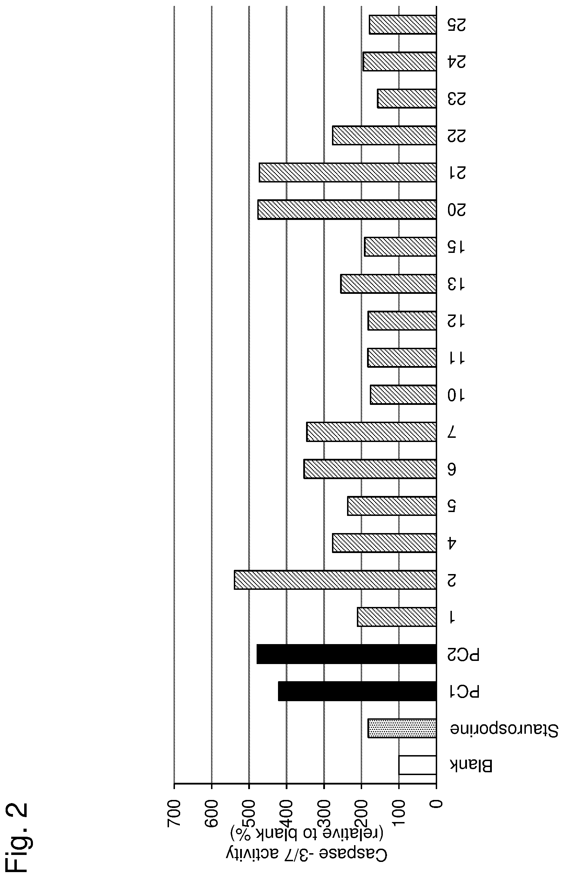

[Figure 2] Figure 2 shows a graph illustrating the influence of the antisense oligonucleotide on the caspase 3/7 activity in the human U251 cells.

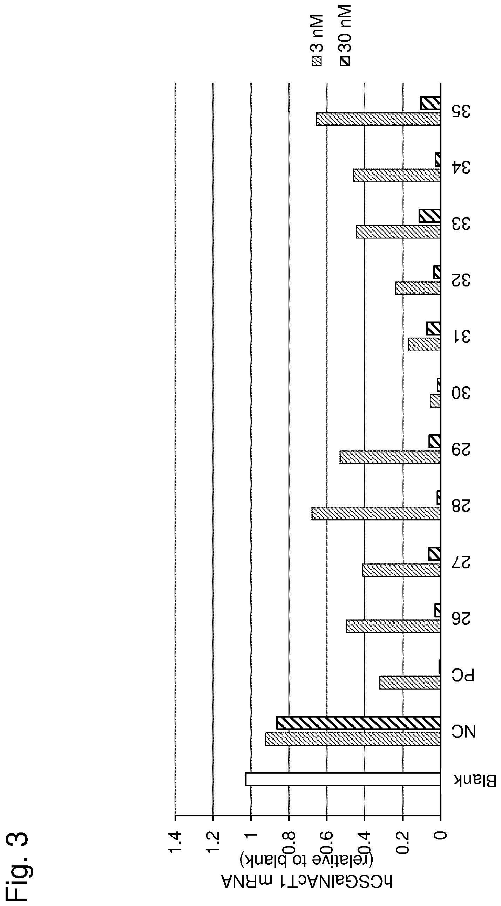

[Figure 3] Figure 3 shows a graph illustrating effects of the antisense oligonucleotide for concentration-dependent expression suppression of the CSGalNAcT1 gene in the human YKG-1 cells.

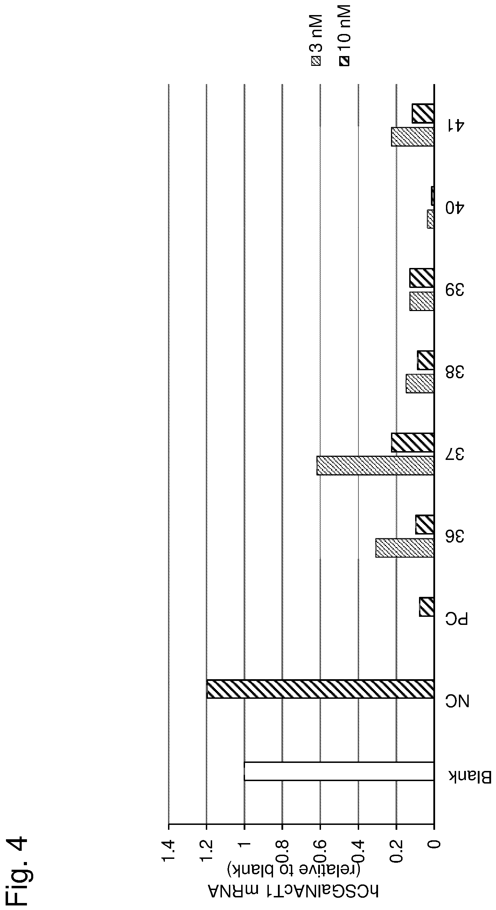

[Figure 4] Figure 4 shows a graph illustrating effects of the antisense oligonucleotide for concentration-dependent expression suppression of the CSGalNAcT1 gene in the human YKG-1 cells.

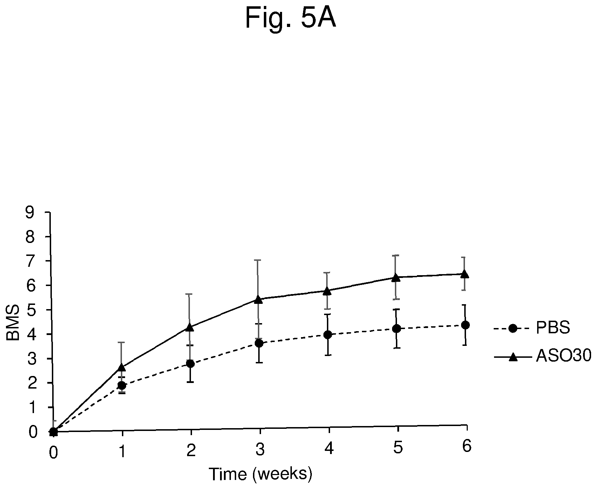

[Figure 5A] Figure 5A shows a graph illustrating the influence of antisense oligonucleotide administration immediately after injury of spinal cord injury model mice on the hindlimb motor function. Figure 5A shows changes in BMS of the groups with the elapse of time. An error bar in the figure indicates a standard deviation.

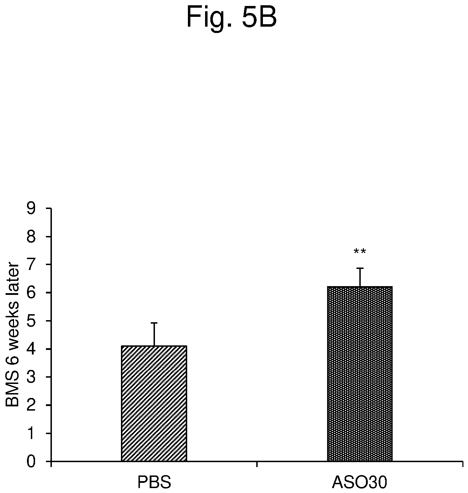

[Figure 5B] Figure 5B shows a graph illustrating the influence of antisense oligonucleotide administration immediately after injury of spinal cord injury model mice on the hindlimb motor function. Figure 5B shows a comparison of BMS of mice in the groups 6 weeks after administration. An error bar in the figure indicates a standard deviation, and "**" indicates that p is less than 0.01 (p < 0.01) in the between-groups t-test.

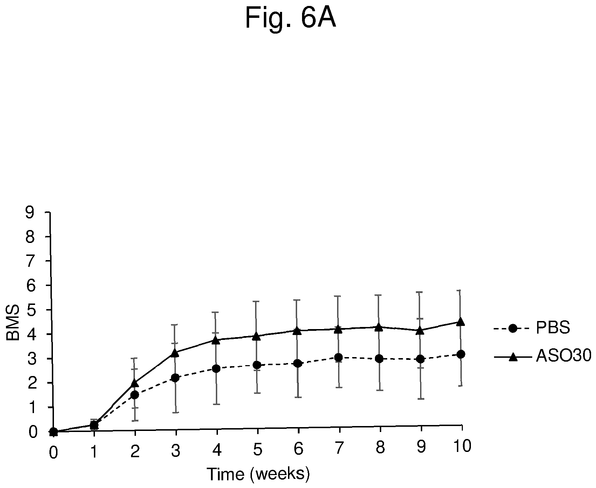

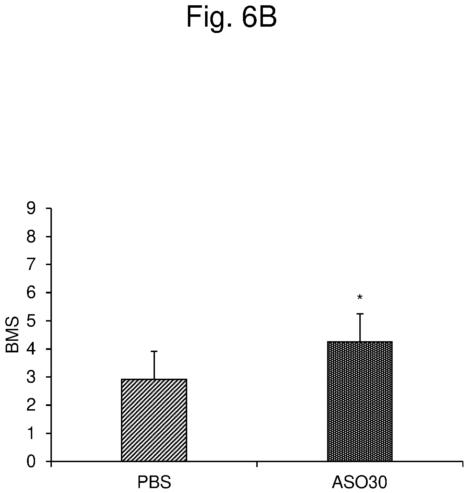

[Figure 6A] Figure 6A shows a graph illustrating the influence of antisense oligonucleotide administration 14 to 16 days after injury of spinal cord injury model mice on the hindlimb motor function. Figure 6A shows changes in BMS of the groups with the elapse of time after the spinal cord injury. The antisense nucleotide or PBS was administered 14 to 16 days after the injury. Figure 6B shows a comparison of BMS of mice in the groups 10 weeks after injury of the spinal cord. An error bar in the figure indicates a standard deviation, and "*" indicates that p is less than 0.05 (p < 0.05) in the between-groups t-test.
[Figure 6B] Figure 6B shows a graph illustrating the influence of antisense oligonucleotide administration 14 to 16 days after injury of spinal cord injury model mice on the hindlimb motor function. Figure 6B shows a comparison of BMS of mice in the groups 10 weeks after injury of the spinal cord. An error bar in the figure indicates a standard deviation, and "*" indicates that p is less than 0.05 (p < 0.05) in the between-groups t-test.

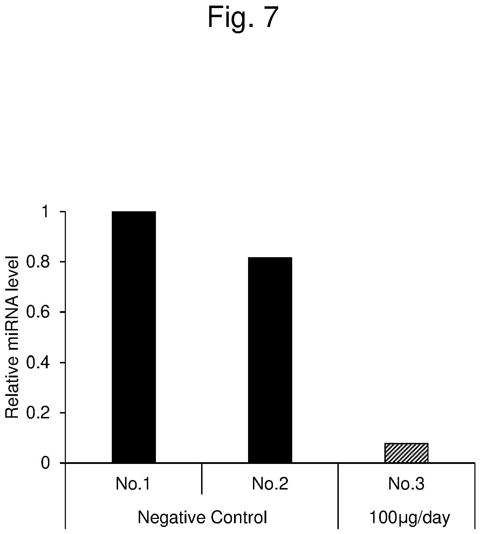

[Figure 7] Figure 7 shows a graph illustrating the CSGalNAcT1 mRNA levels in the spinal cord of spinal cord injury marmoset models to which the antisense oligonucleotide had been administered. The expression level is indicated relative to the expression level of a negative control individual (No. 1), which is designated as 1.

### Description of Embodiments

### 1. Antisense oligonucleotide

The present invention relates to an antisense oligonucleotide. The antisense nucleotide of the present invention suppresses expression of the chondroitin sulfate N-acetylgalactosaminyl-transferase-1 (CSGalNAcT1) gene and has the properties (i) to (iv) described below:
(i) an antisense oligonucleotide is 13- to 25-mer in length;
(ii) an antisense oligonucleotide comprises a gap region, a 3'-wing region bound to the 3' end of the gap region, and a 5'-wing region bound to the 5' end of the gap region;
(iii) nucleotides in the 3'-wing region and in the 5'-wing region include at least one bridged nucleic acid and the bridged nucleic acid is scpBNA or AmNA; and
(iv) an antisense oligonucleotide comprises a nucleotide sequence selected from the group consisting of SEQ ID NOs: 2 to 31 or a sequence derived therefrom by substitution, deletion, or insertion of 1 or 2 nucleic acid bases.

The term "CSGaINAcT1 gene" used herein refer to a gene encoding an enzyme having activity of transferring N-acetylgalactosamine (GalNAc) from UDP-GalNAc to the nonreducing end of glucuronic acid. The CSGalNAcT1 gene was not present in animals (in which central nerve regeneration was observed) before the evolution of the vertebrate phyla, and such gene came into existence after the evolution of animals of the vertebrate phyla, which could have central nerve injury. Origins of the CSGalNAcT1 gene are not particularly limited, and examples of origins include mammals such as primates (e.g., crab-eating monkeys, chimpanzees, and humans) and non-primates (e.g., cattle, pigs, sheep, horses, cats, dogs, guinea pigs, rats, and mice), and more preferably humans. The nucleotide sequence of the CSGalNAcT1 gene can be obtained from the database in the National Center for Biotechnology Information (NCBI). The human CSGalNAcT1 mRNA sequence is shown in SEQ ID NO: 1 (where the sequence of RNA is shown as the sequence of DNA). In the present invention, genes encoding variants of the above-described sequences also fall within the scope of the CSGaNAcT1 gene as long as translated proteins have similar activity.

Herein, the "antisense nucleic acid" or the "antisense oligonucleotide" refers to a single-stranded oligonucleotide including a nucleotide sequence which can hybridize to (that is, complementary to) a part of mRNA of CSGalNAcT1, which is a target gene. In the present invention, in theory, the antisense nucleic acid or the antisense oligonucleotide forms a DNA-RNA hybrid with target mRNA which is cleaved by RNase H, thereby degrading the target mRNA. As a result, expression of the target gene is suppressed. It should be noted that the mechanism is not limited to the theory. The region of mRNA of the target gene which can be hybridized by the antisense oligonucleotide can include 3' UTR, 5' UTR, exon, intron, a coding region, a translation initiation region, a translation termination region, and other nucleic acid regions.

Herein, the "suppression" related to expression of a gene means that the amount (abundance) of mRNA produced by transfer of the gene is reduced. The suppression includes suppression of the amount of mRNA by 20% or more, 30% or more, or 40% or more, preferably 50% or more, and more preferably 80% or more, 90% or more, or 95% or more in comparison with a control. The suppression of gene expression may be determined by any of the methods known in the art, and in particular, the suppression of gene expression can be determined by a method based on PCR such as real-time PCR using cells such as human or mouse cells.

The antisense oligonucleotide of the present invention can reduce the amount of chondroitin sulfate. The reduction of the amount of chondroitin sulfate can be determined by, for example, the In Cell ELISA assay using an anti-chondroitin sulfate antibody. The antisense oligonucleotide of the present invention can reduce the amount of chondroitin sulfate by 10% or more, 20% or more, 30% or more, 40% or more, or 50% or more in comparison with a control.

The term "nucleic acid base" or the "base" used herein refers to a base component of a nucleic acid, and the term refers to a heterocyclic ring moiety, which can be paired with a base of another nucleic acid. The term "nucleotide sequence" used herein refers to a sequence of contiguous nucleic acid bases without taking a nucleic acid-forming sugar, an internucleoside bond, or nucleic acid base modification into consideration.

In general, the "nucleoside" is a combination of a sugar and a nucleic acid base. The "nucleotide" further comprises a phosphate group covalently bound to a sugar moiety of the nucleoside. In general, the phosphate group forms an internucleoside bond of oligonucleotides. The oligonucleotide is formed by covalent bonding of nucleotides adjacent to each other, and it forms a linear polymer oligonucleotide.

The term "modified nucleoside" used herein refers to a nucleoside having a modified sugar and/or a modified nucleic acid base independently. The term "modified nucleotide" refers to a nucleotide having a modified internucleoside bond, a modified sugar, and/or a modified nucleic acid base independently. An oligonucleotide comprising a modified nucleotide is more preferable in comparison with a nonmodified type because of desired properties, such as enhanced affinity for target nucleic acids and enhanced nuclease resistance.

The term "modified internucleoside bond" used herein refers to an internucleoside bond in which a naturally occurring internucleoside bond (that is, phosphodiester bond) is substituted or subjected to some changes. Examples of the modified internucleoside bond include, but are not limited to, a phosphorothioate bond, a phosphorodithioate bond, a phosphorodiamidate bond, and a phosphoramidate bond. The phosphorothioate bond refers to an internucleoside bond in which the non-bridged oxygen atom of a phosphodiester bond is replaced by a sulfur atom. The modified internucleoside bond is preferably a bond having nuclease resistance higher than that of a naturally occurring internucleoside bond.

The term "nonmodified nucleic acid base" or the "natural nucleic acid base" used herein refers to adenine (A) and guanine (G), which are purine bases, thymine (T), cytosine (C), and uracil (U), which are pyrimidine bases, and 5-methylcytosine. In contrast, a nucleic acid base derived from the "nonmodified nucleic acid base" or the "natural nucleic acid base" by artificial modification is referred to as a "modified nucleic acid base." Examples of the modified nucleic acid base include: 5-fluorocytosine, 5-bromocytosine, and 5-iodocytosine; 5-fluorouracil, 5-bromouracil, 5-iodouracil, and 5-hydroxyuracil; 2-thiothymine; N6-methyladenine and 8-bromoadenine; and N2-methylguanine and 8-bromoguanine, although the modified nucleic acid bases are not limited thereto.

The term "modified sugar" used herein refers to a sugar in which a natural sugar moiety of DNA or RNA (that is, a sugar moiety present in DNA (2'-deoxiribose) or RNA (ribose)) is subjected to some changes. Specifically, the term "modified sugar" refers to (1) a molecule in which ribose or 2-deoxyribose is partially substituted with one or more substituents, (2) a pentose or hexose different from ribose or 2-deoxyribose (e.g., a molecule in which either or both of a pentose and a hexose existing in nature, such as hexitol or threose, is (or are) partially substituted with one or more substituents, (3) a molecule in which the entire ribose or 2-deoxyribose or a tetrahydrofuran ring thereof has been substituted with a 5- to 7-membered saturated or unsaturated ring (e.g., cyclohexane, cyclohexene, or morpholine) or a partial structure capable of forming a 5- to 7-membered ring by hydrogen bond (e.g., a peptide structure), or (4) a molecule in which ribose or 2-deoxyribose has been substituted with alkylene glycol having 2 to 6 carbon atoms (e.g., ethylene glycol or propylene glycol). The bicyclic sugar described below is within the scope of the "modified sugar." The modified sugar can impart enhanced affinity for target nucleic acids, enhanced nuclease resistance, and the like to an oligonucleotide. Examples of modified sugars include bicyclic sugars, 5'-vinyl, 5'-methyl, 4'-S, 2'-F, 2'-OCH₃ (2'-methoxy or 2'-O-methyl group), and 2'-O(CH₂)₂OCH₃ substituents.

The term "bicyclic sugar" used herein refers to a sugar having two rings. A nucleic acid comprising a bicyclic sugar moiety is generally referred to as a bridged nucleic acid (BNA). In the bicyclic sugar, a carbon atom at the 2'-position and a carbon atom at the 4'-position may be bridged by two or more atoms. Examples of bridged nucleic acids having bicyclic sugars include, but are not limited to, bridged nucleic acid having a methyleneoxy (4'-CH₂₋O-2') bridge (LNA^{™}, also known as 2',4'-BNA), bridged nucleic acid having an ethyleneoxy (4'-(CH₂)₂₋O-2') bridge (also known as ENA), bridged nucleic acid having a 4'-CH(CH₃)-O-2 bridge (cEt, constrained ethyl), bridged nucleic acid having a 4'-CH(CH₂OCH₃)-O-2' bridge (cMOE, constrained MOE), bridged nucleic acid having an amide bridge (AmNA, amido-bridged nucleic acid), and bridged nucleic acid bearing a spirocyclopropane ring at the bridged moiety (scpBNA, 2'-O,4'-C-spirocyclopropylene bridged nucleic acid). An example of the bridged nucleic acid having an amide bridge is a bridged nucleic acid having a 4'-C(O)-N(CH₃)-2' bridge. Concerning the structures of sugars having an amide bridge and methods for preparing the sugars, see, for example, Yahara, A., et al., Amido-bridged nucleic acids (AmNAs): synthesis, duplex stability, nuclease resistance, and in vitro antisense potency, ChemBioChem., 2012, 13 (7): 2513-2516, Yamamoto, T., et al., Amido-bridged nucleic acids with small hydrophobic residues enhance hepatic tropism of antisense oligonucleotides in vivo, Org. Biomol. Chem., 2015, 13: 3757-3765, and WO 2011/052436. Concerning the sugar having a 4'-CH(CH₃)-O-2' bridge (cEt) and the sugar having a 4'-CH(CH₂OCH₃)-O-2' bridge (cMOE), see Punit, P.S., et al., Short antisense oligonucleotides with novel 2'-4' conformationally restricted nucleoside analogues show improved potency without increased toxicity in animals, J. Med. Chem., 2009, 52 (1): 10-13. Concerning the bridged nucleic acid bearing a spirocyclopropane ring at the bridged moiety (scpBNA), see, for example, Yamaguchi, T., et al., Synthesis and properties of 2'-O,4'-C-spirocyclopropylene bridged nucleic acid (scpBNA), an analogue of 2',4'-BNA/LNA bearing a cyclopropane ring, Chemical Communications, 2015, 51: 973-9740, Horiba, M., et al., Synthesis of scpBNA-mC, -A, and -G Monomers and Evaluation of the Binding Affinities of scpBNA-Modified Oligonucleotides toward Complementary ssRNA and ssDNA, J. Org. Chem., 2016, 81: 11000-11008.

As a bridged nucleic acid, for example, amido-BNA (AmNA) represented by Formula (a) or spirocyclopropylene BNA (scpBNA) represented by Formula (b) can be used.

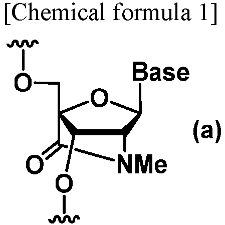

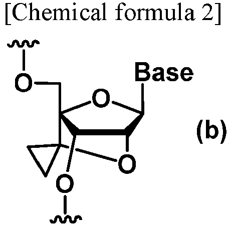

In Formulae (a) and (b) above, "Base" indicates a 5-methylcytosin-1-yl group, thymin-1-yl group, adenin-9-yl group, or guanin-9-yl group, and "Me" indicates methyl. Concerning methods for preparing amido-BNA (AmNA) or spirocyclopropylene BNA (scpBNA), see the method described in WO 2011/052436 or WO 2015/125783.

The antisense oligonucleotide of the present invention is 13- to 25-mer in length, preferably 13- to 17-mer in length, and more preferably 15- to 17-mer in length.

The antisense oligonucleotide of the present invention has a nucleotide sequence selected from the group consisting of SEQ ID NOs: 2 to 31 or a nucleotide sequence derived therefrom by substitution, deletion, or insertion of 1 or 2 nucleic acid bases. The antisense oligonucleotide of the present invention more preferably has a nucleotide sequence selected from the group consisting of SEQ ID NOs: 24, 26, 27, 29, and 31 or a nucleotide sequence derived therefrom by substitution, deletion, or insertion of 1 or 2 nucleic acid bases. The antisense oligonucleotide of the present invention further preferably has the nucleotide sequence represented by SEQ ID NO: 27 or a nucleotide sequence derived therefrom by substitution, deletion, or insertion of 1 or 2 nucleic acid bases.

The antisense oligonucleotide of the present invention is a so-called gapmer comprising a gap region, a 3'-wing region bound to the 3' end of the gap region, and a 5'-wing region bound to the 5' end of the gap region. The term "gapmer" used herein refers to an oligonucleotide comprising a central region comprising at least four contiguous deoxyribonucleosides (a DNA gap region) and regions positioned on the 5'-end side and the 3'-end side of the central region and comprising an unnatural nucleoside (a 5'-wing region and a 3'-wing region). The length of the DNA gap region is preferably 7- to 17-mer, more preferably 7- to 16-mer, 7- to 14-mer, 7- to 12-mer, or 8- to 10-mer, and further preferably 9-mer. The length of the 5'-wing region and that of the 3'-wing region are each preferably 2- to 6-mer, 2-to 5-mer, or 2- to 4-mer. The length of the 5'-wing region is more preferably 3-mer. The length of the 3'-wing region is more preferably 3-mer. The length of the 5'-wing region, that of the DNA gap region, and that of the 3'-wing region are most preferably 3-mer, 9-mer, and 3-mer, respectively. The 5'-wing region and the 3'-wing region are only required to comprise at least one unnatural nucleoside, and these regions may comprise a natural nucleoside.

The antisense oligonucleotide of the present invention comprises at least one amido-BNA (AmNA) represented by the formula (a) above or spirocyclopropylene BNA (scpBNA) represented by the formula (b) above as a bridged nucleic acid in the nucleotides in the 5'-wing region and in the 3'-wing region. All the nucleosides in the 5'-wing region and in the 3'-wing region may be bridged nucleic acids. The term "bridged nucleic acid" used herein specifically refers to a nucleic acid having a sugar having a bicyclic sugar. It is preferable that the antisense oligonucleotide of the present invention comprise at least one scpBNA in the 5'-wing region and/or in the 3'-wing region. The antisense oligonucleotide of the present invention preferably comprises at least one scpBNA, preferably in the 3'-wing region, more preferably at the 3'-end. It is the most preferable that all the nucleosides in the 5'-wing region be AmNA. It is the most preferable that, in the 3'-wing region, nucleosides at the 3'-end thereof be scpBNA and all other nucleosides be AmNA. The 5'-wing region and/or the 3'-wing region may comprise natural nucleosides. The natural nucleosides may be methylated herein. Specifically, the natural nucleoside may include 5-methylcytosine.

In the present invention, the antisense oligonucleotide may comprise a mimic nucleotide such as a peptide nucleic acid or a morpholino nucleic acid.

Different nucleotides in the same chain of the antisense oligonucleotide of the present invention may be subjected to different modifications independently. In order to enhance nuclease resistance, for example, identical nucleotides may have a modified internucleoside bond (for example, a phosphorothioate bond). In the antisense oligonucleotide of the present invention, in particular, at least one internucleoside bond is preferably a phosphorothioate bond. At least 70%, at least 80%, or at least 90% of the internucleoside bonds in the antisense oligonucleotide may be phosphorothioate bonds.

When the antisense oligonucleotide of the present invention is a gapmer, at least one internucleotide bond in the 3'-wing region and in the 5'-wing region is preferably a phosphodiester bond. Of the internucleoside bonds in the wing region, in addition, the proportion of phosphodiester (PO) bonds relative to the total of phosphorothioate (PS) bonds and PO bonds (PO/(PS+PO)) may be less than 0.5, in particular, less than 0.4, or less than 0.3.

The antisense oligonucleotide of the present invention can have any of the structures shown in Tables 1 to 3, although the structure is not limited thereto. In the tables, the target sequence indicates the position from the 5' end of the nucleotide sequence represented by SEQ ID NO: 1. A lowercase letter indicates an unmodified nucleotide, an uppercase letter indicates a bridged nucleic acid, "(Am)" indicates AmNA, "(Sc)" indicates scpBNA, and "5" indicates 5-methylcytosine. The symbol "^" indicates a phosphorothioate bond. In the tables, "SEQ ID NO:" indicates a sequence identification number of a nucleotide sequence without oligonucleotide modification.

**[Table 1]**

| No. | Oligonucleotide name | ID No. | Antisense (5' → 3') | Target sequence | | SEQ ID NO: |
|---|---|---|---|---|---|---|
| | | | | 5' end | 3' end | |
| 1 | hCSGALNACT1-1483-AmNA(15) | LX-A1011 | 5(Am)^T(Am)^G(Am)^c^a^t^a^c^t^c^t^g^T(Am)^G(Am)^g | 1483 | 1497 | 2 |
| 2 | hCSGALNACT1-1484-AmNA(15) | LX-A1012 | G(Am)^5(Am)^T(Am)^g^c^a^t^a^c^t^c^t^G(Am)^T(Am)^g | 1484 | 1498 | 3 |
| 3 | hCSGALNACT1-1485-AmNA(15) | LX-A1013 | T(Am)^G(Am)^5(Am)^t^g^c^a^t^a^c^t^c^T(Am)^G(Am)^t | 1485 | 1499 | 4 |
| 4 | hCSGALNACT1-1508-AmNA(15) | LX-A1016 | A(Am)^G(Am)^A(Am)^g^t^a^a^a^g^c^t^a^T(Am)^5(Am)^g | 1508 | 1522 | 5 |
| 5 | hCSGALNACT1-1509-AmNA(15) | LX-A1017 | T(Am)^A(Am)^G(Am)^a^g^t^a^a^a^g^c^t^A(Am)^T(Am)^c | 1509 | 1523 | 6 |
| 6 | hCSGALNACT1-1510-AmNA(15) | LX-A1018 | G(Am)^T(Am)^A(Am)^g^a^g^t^a^a^a^g^c^T(Am)^A(Am)^t | 1510 | 1524 | 7 |
| 7 | hCSGALNACT1-1511-AmNA(15) | LX-A1019 | T(Am)^G(Am)^T(Am)^a^g^a^g^t^a^a^a^g^5(Am)^T(Am)^a | 1511 | 1525 | 8 |
| 8 | hCSGALNACT1-1612-AmNA(15) | LX-A1028 | A(Am)^G(Am)^G(Am)^c^t^g^a^t^t^c^a^a^T(Am)^G(Am)^g | 1612 | 1626 | 9 |
| 9 | hCSGALNACT1-1615-AmNA(15) | LX-A1029 | 5(Am)^5(Am)^A(Am)^a^g^g^c^t^g^a^t^t^5(Am)^A(Am)^a | 1615 | 1629 | 10 |
| 10 | hCSGALNACT1-1845-AmNA(15) | LX-A1045 | A(Am)^A(Am)^5(Am)^a^t^t^g^a^t^a^a^g^5(Am)^G(Am)^t | 1845 | 1859 | 11 |
| 11 | hCSGALNACT1-1846-AmNA(15) | LX-A1046 | T(Am)^A(Am)^A(Am)^c^a^t^t^g^a^t^a^a^G(Am)^5(Am)^g | 1846 | 1860 | 12 |
| 12 | hCSGALNACT1-1847-AmNA(15) | LX-A1047 | A(Am)^T(Am)^A(Am)^a^c^a^t^t^g^a^t^a^A(Am)^G(Am)^c | 1847 | 1861 | 13 |
| 13 | hCSGALNACT1-1849-AmNA(15) | LX-A1048 | 5(Am)^G(Am)^A(Am)^t^a^a^c^a^t^t^g^a^T(Am)^A(Am)^a | 1849 | 1863 | 14 |
| 14 | hCSGALNACT1-1863-AmNA(15) | LX-A1054 | T(Am)^T(Am)^T(Am)^t^g^c^t^a^g^a^g^g^5(Am)^A(Am)^c | 1863 | 1877 | 15 |
| 15 | hCSGALNACT1-2225-AmNA(15) | LX-A1076 | G(Am)^G(Am)^A(Am)^t^t^g^t^a^c^t^g^a^5(Am)^T(Am)^g | 2225 | 2239 | 16 |
| 16 | hCSGALNACT1-2226-AmNA(15) | LX-A1077 | A(Am)^G(Am)^G(Am)^a^t^t^g^t^a^c^t^g^A(Am)^5(Am)^t | 2226 | 2240 | 17 |
| 17 | hCSGALNACT1-2227-AmNA(15) | LX-A1078 | 5(Am)^A(Am)^G(Am)^g^a^t^t^g^t^a^c^t^G(Am)^A(Am)^c | 2227 | 2241 | 18 |
| 18 | hCSGALNACT1-2228-AmNA(15) | LX-A1079 | 5(Am)^5(Am)^A(Am)^g^g^a^t^t^g^t^a^c^T(Am)^G(Am)^a | 2228 | 2242 | 19 |
| 19 | hCSGALNACT1-2230-AmNA(15) | LX-A1080 | T(Am)^G(Am)^5(Am)^c^a^g^g^a^t^t^g^t^A(Am)^5(Am)^t | 2230 | 2244 | 20 |
| 20 | hCSGALNACT1-2329-AmNA(15) | LX-A1090 | A(Am)^5(Am)^G(Am)^t^c^a^t^c^c^c^a^a^A(Am)^T(Am)^c | 2329 | 2343 | 21 |
| 21 | hCSGALNACT1-2330-AmNA(15) | LX-A1091 | 5(Am)^A(Am)^5(Am)^g^t^c^a^t^c^c^c^a^A(Am)^A(Am)^t | 2330 | 2344 | 22 |
| 22 | hCSGALNACT1-2444-AmNA(15) | LX-A1101 | A(Am)^5(Am)^5(Am)^a^c^t^a^t^g^a^g^g^T(Am)^T(Am)^g | 2444 | 2458 | 23 |
| 23 | hCSGALNACT1-3743-AmNA(15) | LX-A1191 | G(Am)^A(Am)^T(Am)^t^g^t^t^t^g^g^t^t^5(Am)^A(Am)^c | 3743 | 3757 | 24 |
| 24 | hCSGALNACT1-3744-AmNA(15) | LX-A1192 | A(Am)^G(Am)^A(Am)^t^t^g^t^t^t^g^g^t^T(Am)^5(Am)^a | 3744 | 3758 | 25 |
| 25 | hCSGALNACT1-3745-AmNA(15) | LX-A1193 | G(Am)^A(Am)^G(Am)^a^t^t^g^t^t^t^g^g^T(Am)^T(Am)^c | 3745 | 3759 | 26 |

**[Table 2]**

| No. | Oligonucleotide name | ID No. | Antisense (5' → 3') | Target sequence | | SEQ ID NO: |
|---|---|---|---|---|---|---|
| | | | | 5' end | 3' end | |
| 26 | hCSGALNACT1-1845-AmNA, SCP(15) | LX-A1885 | A(Am)^A(Am)^5(Am)^a^t^t^g^a^t^a^a^g^5(Am)^G(Am)^T(Sc) | 1845 | 1859 | 11 |
| 27 | hCSGALNACT1-1845-AmNA, SCP, PO(15) | LX-A1886 | A(Am)A(Am)5(Am)a^t^t^g^a^t^a^a^g^5(Am)G(Am)T(Sc) | 1845 | 1859 | 11 |
| 28 | hCSGALNACT1-1847-AmNA, SCP(15) | LX-A1887 | A(Am)^T(Am)^A(Am)^a^c^a^t^t^g^a^t^a^A(Am)^G(Am)^5(Sc) | 1847 | 1861 | 13 |
| 29 | hCSGALNACT1-1847-AmNA, SCP, PO(15) | LX-A1888 | A(Am)T(Am)A(Am)a^c^a^t^t^g^a^t^a^A(Am)G(Am)5(Sc) | 1847 | 1861 | 13 |
| 30 | hCSGALNACT1-3743-AmNA, SCP(15) | LX-A1897 | G(Am)^A(Am)^T(Am)^t^g^t^t^t^g^g^t^t^5(Am)^A(Am)^5(Sc) | 3743 | 3757 | 27 |
| 31 | hCSGALNACT1-3743-AmNA, SCP, PO(15) | LX-A1898 | G(Am)A(Am)T(Am)t^g^t^t^t^g^g^t^t^5(Am)A(Am)5(Sc) | 3743 | 3757 | 27 |
| 32 | hCSGALNACT1-3745-AmNA, SCP(15) | LX-A1899 | G(Am)^A(Am)^G(Am)^a^t^t^g^t^t^t^g^g^T(Am)^T(Am)^5(Sc) | 3745 | 3759 | 26 |
| 33 | hCSGALNACT1-3745-AmNA, SCP, PO(15) | LX-A1900 | G(Am)A(Am)G(Am)a^t^t^g^t^t^t^g^g^T(Am)T(Am)5(Sc) | 3745 | 3759 | 26 |
| 34 | hCSGALNACT1-2446-AmNA, SCP(15) | LX-A1901 | G(Am)^T(Am)^A(Am)^c^c^a^c^t^a^t^g^a^G(Am)^G(Am)^T(Sc) | 2446 | 2460 | 28 |
| 35 | hCSGALNACT1-2446-AmNA, SCP, PO(15) | LX-A1902 | G(Am)T(Am)A(Am)c^c^a^c^t^a^t^g^a^G(Am)G(Am)T(Sc) | 2446 | 2460 | 28 |

**[Table 3]**

| No. | Oligonucleotide name | ID No. | Antisense (5' → 3') | Target sequence | | SEQ ID NO: |
|---|---|---|---|---|---|---|
| | | | | 5' end | 3' end | |
| 36 | hCSGALNACT1-1483-AmNA, PO(15) | LX-A2957 | 5(Sc)T(Am)G(Am)c^a^t^a^c^t^c^t^g^T(Am)G(Am)^g | 1483 | 1497 | 2 |
| 37 | hCSGALNACT1-3744-AmNA, PO(15) | LX-A2962 | A(Am)G(Am)A(Am)t^t^g^t^t^t^g^g^t^T(Am)5(Am)^a | 3744 | 3758 | 25 |
| 38 | hCSGALNACT1-3743-AmNA, SCP, PO(15) | LX-A2996 | G(Am)^A(Am)T(Am)^t^g^t^t^t^g^g^t^t^5(Am)A(Am)^5(Sc) | 3743 | 3757 | 24 |
| 39 | hCSGALNACT1-3743-AmNA, SCP, PO(17) | LX-A2997 | G(Am)^A(Am)G(Am)^a^t^t^g^t^t^t^g^g^t^t^5(Am)A(Am)^5(Sc) | 3743 | 3759 | 29 |
| 40 | hCSGALNACT1-1481-AmNA, SCP, PO(17) | LX-A2998 | 5(Sc)^T(Am)G(Am)^c^a^t^a^c^t^c^t^g^t^g^g^5(Am)5(Sc) | 1481 | 1497 | 30 |
| 41 | hCSGALNACT1-2328-AmNA, SCP, PO(17) | LX-A2999 | 5(Am)^A(Am)5(Am)^g^t^c^a^t^c^c^c^a^a^a^t^5(Am)5(Sc) | 2328 | 2344 | 31 |

The antisense oligonucleotide of the present invention can be produced by a method known in the art. The antisense oligonucleotide can be produced by, for example, synthesis using a commercially available automated oligonucleotide synthesizer, followed by purification using a reversed phase column or the like. Alternatively, the antisense oligonucleotide can be obtained by specifying a nucleotide sequence, a modified moiety, and a type, and ordering from a manufacturer (for example, GeneDesign, Inc.).

The antisense oligonucleotide of the present invention suppresses expression of the CSGalNAcT1 gene to inhibit biosynthesis of chondroitin, and as a result, the amount of chondroitin sulfate can be reduced. An increase in chondroitin sulfate after injury of the spinal cord inhibits axon regeneration, and it hinders recovery from the spinal cord injury. A spinal cord injury can be treated (or recovery from a spinal cord injury can be promoted) by reducing the amount of chondroitin sulfate using the antisense oligonucleotide of the present invention. On the other hand, heparan sulfate has been reported to promote axon regeneration (Takeuchi, K., *et al*., 2013, described above). The antisense oligonucleotide of the present invention can reduce the amount of chondroitin sulfate without reducing the amount of heparan sulfate, so that the spinal cord injury can be effectively treated.

### 2. Pharmaceutical composition

The pharmaceutical composition of the present invention comprises the antisense oligonucleotide of the present invention. The pharmaceutical composition of the present invention can be used for treating a disease or condition related to an increase in chondroitin sulfate.

Examples of the disease or condition related to an increase in chondroitin sulfate include, but are not limited to, spinal cord injury, cerebral vascular disorders (for example, cerebral injury and cerebral ischemia), digestive system diseases (for example, inflammatory colitis), and cutaneous injury or inflammation.

The term "spinal cord injury" used herein refers to a pathological state in which the spinal cord is injured. The cause of a spinal cord injury does not matter. A spinal cord injury may be a traumatic spinal cord injury. The spinal cord injury includes a complete spinal cord injury and an incomplete spinal cord injury. The complete spinal cord injury refers to a state in which the spinal cord is transversely discontinued. The incomplete spinal cord injury refers to a state in which the spinal cord is partially injured. The spinal cord injury develops various disorders, such as motor function disorders, sensory disorders, and autonomic nerve disorders.

Herein, the "treatment" can include alleviation or cure of symptoms (for example, motor function disorders) caused by a spinal cord injury in subjects having a spinal cord injury.

Scars formed as a result of restoration of tissues after injury of the spinal cord are generally considered to inhibit regeneration of nerves. Thus, recovery from the spinal cord injury can be promoted by suppressing formation of scars. The pharmaceutical composition according to the present invention may be used for suppressing or reducing formation of scars after injury of the spinal cord.

The pharmaceutical composition may further comprise any pharmaceutical aid that is usually used in the field of pharmaceutical production. Herein, examples of the pharmaceutical aid that can be used include various carriers and additives such as pharmaceutically acceptable carriers (solid or liquid carriers), excipients, stabilizers, disintegrating agents, surfactants, binding agents, lubricants, emulsifiers, suspensions, antioxidants, fragrances, fillers, solubilizing agents, coating agents, colorants, flavoring agents, preservatives, and buffers. Specific examples of the pharmaceutical aid include water, physiological saline, other aqueous solvents, pharmaceutically acceptable organic solvents, mannitol, lactose, starch, microcrystalline cellulose, glucose, calcium, polyvinyl alcohol, collagen, polyvinyl pyrrolidone, carboxyvinyl polymers, sodium alginate, water-soluble dextran, water-soluble dextrin, carboxymethyl starch sodium, pectin, gum Arabic, xanthan gum, casein, gelatin, agar, propylene glycol, polyethylene glycol, petroleum jerry, paraffin, glycerin, stearyl alcohol, stearic acid, and sorbitol. The pharmaceutical aids can be selected appropriately or in combination according to the dosage form of a preparation.

The pharmaceutical composition can be orally or parenterally administered to a subject. Examples of parenteral administration include, but are not limited to, intravenous administration, subcutaneous administration, intramedullary administration, and local administration. It is preferable to locally administer the pharmaceutical composition directly to the site of injury in order to achieve efficient treatment effects. Further, the pharmaceutical composition can be continuously administered to the site of injury using a continuous injection pump. Further, the pharmaceutical composition may be retained on the site of injury with the pharmaceutical composition carried in a sponge. The pharmaceutical composition may be formed into preparations such as injections and drops. Those skilled in the art can produce these preparations by conventional methods.

The pharmaceutical composition may be administered in a therapeutically effective amount. The specific dosage of the pharmaceutical composition is determined for each subject at the discretion of, for example, a physician, depending on the patient's severity, systemic health condition, age, sex, weight, tolerance to treatment, and the like. For example, the pharmaceutical composition may be administered in an amount such that the dosage of the antisense oligonucleotide is 0.000001 mg/kg of body weight/day to 1000 mg/kg of body weight/day, or 0.001 mg/kg of body weight/day to 1 mg/kg of body weight/day. The pharmaceutical composition can be administered in a single dose or a plurality of doses, and for example, the pharmaceutical composition may be administered to the subject several times or some dozen times at fixed time intervals, for example, at intervals of 1 day, 2 days, 3 days, 4 days, 5 days, 6 days, 1 week, 2 weeks, 3 weeks, 1 month, or the like. Alternatively, the pharmaceutical composition may be continuously administered using a continuous injection pump as described above. When the pharmaceutical composition is continuously administered, the dosage (rate), the period, and the like can be appropriately set by those skilled in the art.

When a pharmaceutical composition is used for treating a spinal cord injury by itself, the pharmaceutical composition may be administered at any of the acute phase, the subacute phase, or the chronic phase. The pharmaceutical composition is preferably administered at the acute phase or the subacute phase, and it is more preferably administered at the subacute phase. By administering the pharmaceutical composition at the acute phase or the subacute phase, desired effects of the pharmaceutical composition are achieved more effectively. Concerning the spinal cord injury, the "acute phase" indicates a phase at which injury-induced inflammation takes place, the "subacute phase" indicates a period from the settlement of inflammation to the fixation of the glial scar, and the "chronic phase" indicates a period after the fixation of the glial scar.

The subjects to which the pharmaceutical composition is to be administered are mammals, such as primates (for example, crab-eating monkeys, chimpanzees, and humans) and non-primates (for example, cattle, pigs, sheep, horses, cats, dogs, guinea pigs, rats, and mice), and more preferably humans. The subject may be a subject having a spinal cord injury.

The present invention also provides a method for treating a disease or condition related to an increase in chondroitin sulfate, which comprises administering an antisense oligonucleotide or a pharmaceutical composition according to an embodiment of the present invention to a subject who is in need of treatment.

The present invention also provides use of an antisense oligonucleotide according to an embodiment of the present invention in the production of a pharmaceutical product used for treating a disease or condition related to an increase in chondroitin sulfate.

### Examples

Hereafter, the present invention is described in greater detail with reference to the examples, although the technical scope of the present invention is not limited to these examples.

### (Example 1) Synthesis of antisense oligonucleotide

As a sugar-modified nucleoside, amido-BNA (AmNA) or spirocyclopropylene BNA (scpBNA) was used. The bridged nucleic acid used in the present invention; i.e., amido-BNA (AmNA) or spirocyclopropylene BNA (scpBNA), was synthesized with reference to the method described in International Publication WO2011/052436 or WO2015/125783. The oligonucleotide associated with the present invention was synthesized in accordance with the method described in, for example, Tetrahedron Letters 22, 1859-1862, 1981 or International Publication WO 2011/052436. Specifically, the oligonucleotide was synthesized in the manner described below.

An oligonucleotide comprising amido-BNA (AmNA) or spirocyclopropylene BNA (scpBNA) was synthesized using an automated oligonucleotide synthesizer (ns-8, GeneDesign, Inc.) on a 0.2-µmol scale or a 1.0-µmol scale. Chain elongation was performed according to a conventional phosphoramidite protocol (solid carriers: CPG, iodine was used for oxidation to form the phosphodiester (PO) skeleton, DDTT (((dimethylamino-methylidene)amino)-3H-1,2,4-dithiazaoline-3-thione) was used for sulfuration to form the phosphorothioated (PS) skeleton). An oligonucleotide comprising amido-BNA (AmNA) or spirocyclopropylene BNA (scpBNA) with a hydroxyl group not protected with a DMTr (4,4'-dimethoxytrityl) group at the 5' position at the end and supported on a solid phase at the 3' position was synthesized. Subsequently, bases were treated to cleave the target from the solid support, the solvent was removed by distillation, and the resulting crude product was purified by reverse phase HPLC to obtain a target oligonucleotide. The purity and the structure of the oligonucleotides obtained were examined by LC-MS (Waters Corporation).

An antisense oligonucleotide was designed to target mRNA of human CSGALNACT1 (hCSGalNAcT1) (GenBank: NM_001130518.1 (SEQ ID NO: 1)).

In order to select a target region, a loop structure or the like, which is a region to which an antisense oligonucleotide can easily access, was selected based on the prediction of the secondary structure of mRNA, the thermodynamically stable complementary bond of which had been calculated based on the primary sequence of mRNA. The reverse complementary sequence (antisense sequence) of mRNA was selected in the region, and a sequence comprising an antisense CG sequence expressing toxicity was excluded from the sequence indicated above. Homology between the sequence of interest and the gene other than hCSGalNAcT1 was evaluated using GGGenome (GGGenome:gggenome.dbcls.jp/) to select a candidate sequence.

An oligonucleotide consisting of a nucleotide sequence complementary to the candidate sequence selected in the manner described above was designed as an antisense oligonucleotide. The antisense oligonucleotide was designed as a 15-mer, artificial nucleic acid regions comprising sugar-modified nucleosides were provided at the 5' end and the 3' end, and a natural nucleic acid region comprising natural nucleosides (DNA) was provided at the center. More specifically, an oligonucleotide was designed as a 3-9-2-1 gapmer comprising 3 sugar-modified nucleosides on the 5' terminal side (the 5'-wing region), subsequent 9 natural nucleosides (DNAs) (the gap region), 2 sugar-modified nucleosides out of the 3 bases on the 3' terminal side (the 3'-wing region), and a DNA at the 3' end.

The designed and prepared antisense oligonucleotides are shown in Tables 1, 2, and 3. Tables 1 to 3 show the names of antisense oligonucleotides ("Oligonucleotide name") in combination with the 5' ends and the 3' ends (positions are shown on the basis of SEQ ID NO: 1) of the sequences of the target regions thereof. For example, "hCSGALNACT1-1483-AmNA(15)" indicates a 15-mer antisense oligonucleotide comprising AmNA, which targets a region starting from the 1483rd nucleotide of the nucleotide sequence represented by SEQ ID NO: 1 as the 5' end.

In Tables 1 to 3, "5" indicates 5-methylcytosine (mC), A (Am), G (Am), 5 (Am), and T (Am) indicate AmNA nucleotides, A (Sc), G (Sc), 5 (Sc), and T (Sc) indicate ScpBNA nucleotides, a, g, c, and t indicate unmodified nucleotides (DNAs), and the symbol "^" indicates a phosphorothioate bond.

In Tables 1 to 3, each antisense oligonucleotide is indicated as "hCSGALNACT1-p-n(L)," wherein "p" indicates the number of the nucleotide position corresponding to the 5' end of the target region in SEQ ID NO: 1, "n" indicates a sugar-modified nucleoside (artificial nucleic acid) ("AmNA" in Tables 1 to 3), and "L" indicates a length of an antisense oligonucleotide. In the case of "hCSGALNACT1-1483-AmNA(15)," for example, the 1483rd nucleotide in the nucleotide sequence represented by SEQ ID NO: 1 is the 5' end of the target region comprising AmNA, and the base length is 15. In the case of "hCSGALNACT1-1845-AmNA,SCP,PO(15)," for example, the 1845th nucleotide in the nucleotide sequence represented by SEQ ID NO: 1 is the 5' end of the target region, the sequence comprises AmNA·SCP in which the phosphorothioate bond has been converted to the PO bond, and the base length is 15. When the sequence of the antisense oligonucleotide is indicated in the 5'-to-3'-direction (i.e., 5' -> 3'), a 15-mer antisense oligonucleotide indicated as "hCSGalNAcT1 - 1483" indicates, for example, a sequence extended by 15 bases from the 1483rd nucleotide toward the 3' end of the nucleotide sequence represented by SEQ ID NO: 1; i.e., an antisense oligonucleotide designed as a nucleotide sequence (5'-ctgcatactctgtgg-3') (SEQ ID NO: 2) complementary to 5'-ccacagaguaugcag-3', which is a target mRNA sequence based on 5'-ccacagagtatgcag-3', which is a DNA nucleotide sequence comprising a region from the 1483rd nucleotide to the 1497th nucleotide of SEQ ID NO: 1.

The nucleotide sequence (5'-ctgcatactctgtgg-3') (SEQ ID NO: 2) of the thus-designed antisense oligonucleotide hCSGALNACT1-1483-AmNA(15) is complementary to the nucleotide sequence (5'-ccacagagtatgcag-3') of the region from the 1483rd nucleotide to the 1497th nucleotide of SEQ ID NO: 1.

### (Example 2) Suppression of hCSGalNAcT1 mRNA expression in human glioblastoma cell line in vitro

Concerning the antisense oligonucleotide prepared in Example 1, activity of suppressing hCSGalNAcT1 mRNA expression in a human glioblastoma cell line was inspected *in vitro.* A sample not treated with an oligonucleotide was designated as a blank sample. For comparison, an oligonucleotide of a negative control (NC) comprising LNA:
G(L)^A(L)^A(L)^a^a^c^t^a^a^a^a^t^G(L)^A(L)^g (SEQ ID NO: 32); and an oligonucleotide of a positive control (PC) comprising LNA:
A(L)^5(L)^A(L)^t^c^a^c^a^g^a^a^a^A(L)^A(L)^g (SEQ ID NO: 33), were used, wherein symbols are as defined above with respect to Tables 1 to 3, except that "(L)" indicates LNA. NC is identical to the oligonucleotide used as the "nucleic acid NEG#1 (SEQ ID NO: 186)" in Patent Literature 1. PC is the oligonucleotide used as "hT1-2126-LNA(15) (SEQ ID NO: 113)" in Patent Literature 1, which is identical to the oligonucleotide verified to inhibit CSGALNACT1.

As a human glioblastoma cell line, the U251-MG cells (European Collection of Authenticated Cell Cultures: ECACC) were used. The U251-MG cells were transfected with the antisense oligonucleotides using a commercially available transfection reagent (Lipofectamine 3000, ThermoFisher Scientific), the mRNA expression levels were measured by qRT-PCR, and the knockdown activity (suppression of mRNA expression) was inspected. The procedure is described below. The U251-MG cells at the logarithmic growth phase were seeded on a 24-well plate (the plate comprises the Eagle's minimum essential medium (EMEM) comprising 10% fetal bovine serum (FBS) and 1% non-essential amino acid) at 4.0 × 10⁴ cells/well. The antisense oligonucleotides were added to the wells to the final concentration of 100 nM 24 hours thereafter, followed by incubation for 24 hours. Thereafter, the cells were collected, and total RNA was extracted using the RNA extraction reagent (Maxwell RSC simply RNA Cells Kit, Promega) and the equipment (Maxwell RSC Instrument, Promega). cDNA was synthesized using the total RNA as a template and a reverse transcriptase (GoScript Reverse Transcriptase System, Promega). Reverse transcription was performed with a thermal cycle of 25°C for 5 minutes, 42°C for 60 minutes, 70°C for 15 minutes, and 4°C for 5 minutes. PCR amplification was performed using the synthesized cDNA as a template and a reagent for nucleic acid amplification (GoTaq qPCR Master Mix, Promega). The nucleic acid amplification reaction was performed with a thermal cycle of 95°C for 2 minutes, followed by 40 to 45 cycles of 95°C for 5 seconds and 60°C for 30 seconds. In real-time PCR, the mRNA level of the human glyceraldehyde-3-phosphate dehydrogenase (hGAPDH) of the house keeping gene was simultaneously quantified, and the mRNA level of hCSGALNACT1 relative to the mRNA level of hGAPDH was evaluated. The mRNA levels of the respective antisense oligonucleotides are shown relative to the mRNA level of the cells not treated with the oligonucleotides, which is designated as 1. The test was performed by dual measurement (n = 2) under each condition, and the average was determined.

The sets of primers used are as indicated below:
(Primers for detecting hCSGALNACT1; FASMAC)
   hCSGalNAcT1 Set1-Fw:
      5'-TCAGGGAGATGTGCATTGAG-3' (SEQ ID NO: 34)
   hCSGalNAcT1 Set1-Rv:
      5'-AGTTGGCAGCTTTGGAAGTG-3' (SEQ ID NO: 35)
   hCSGalNAcT1 Set2-Fw:
      5'-GGAGACCCTGAACAGTCCTG-3' (SEQ ID NO: 36)
   hCSGalNAcT1 Set2-Rv:
      5'-GCCGTTTGAATTCGTGTTTG-3' (SEQ ID NO: 37)
(Primers for detecting hGAPDH; FASMAC)
   Fw: 5'-GAGTCAACGGATTTGGTCGT-3' (SEQ ID NO: 38)
   Rv: 5'-GACAAGCTTCCCGTTCTCAG-3' (SEQ ID NO: 39)

Figure 1 shows the results of analysis of suppression of mRNA expression. In Figure 1, the numbers on the horizontal axis correspond to the numbers (Nos.) of the antisense oligonucleotides shown in Tables 1 to 3. With the addition of the antisense oligonucleotide, larger number of cells exhibited lowered mRNA levels, compared with the cells not treated with the oligonucleotides (blank cells) and the cells treated with NC. Specifically, a large number of antisense oligonucleotides suppressing mRNA expression were found.

### (Example 3) Analysis of cytotoxicity of antisense oligonucleotide on human uterine cervix cancer cells in vitro

Concerning the 51 types of antisense oligonucleotides exhibiting the high knockdown activity (suppression of mRNA expression) in Example 2 above and high homology in the gene sequences between humans and mice, the influence thereof on the activity of an apoptosis marker Caspase-3/7 was inspected in order to evaluate the *in vitro* cytotoxicity of the antisense oligonucleotides in human cell lines. The samples not treated with the oligonucleotides were designated as blank samples. For comparison, two types of positive controls comprising staurosporine and AmNA or LNA: PC1 and PC2 oligonucleotides, were used:
A(Am)^T(Am)^5(Am)^a^t^g^g^c^t^g^c^a^g^5(Am)^T(Am)^t (PC1, SEQ ID NO: 40); and
G(L)^T(L)^5(L)^a^t^c^c^c^a^a^a^t^5(L)^5(L)^a (PC2, SEQ ID NO: 41),
wherein the symbols are as defined above with respect to Tables 1 to 3, except that "(L)" indicates LNA. PC1 is a sequence prepared by substituting LNA in the sequence used in Burel et al., Nucleosides, Nucleotides, and Nucleic Acids 44, 2093-2109, 2015 (Isis No.569717, PTEN) with AmNA and substituting the 3' end with DNA, and such sequence was verified to exert hepatotoxicity in the literature. PC2 is the oligonucleotide used as "hT1-2327-LNA(15) (SEQ ID NO: 35)" in Patent Literature 1, and PC2 is identical to the sequence that was verified to inhibit CSGALNACT1. Both the PC1 and PC2 sequences were used as positive controls to increase the caspase concentration.

As human uterine cervix cancer cells, the HeLa-S3 cells were used. The HeLa-S3 cells were transfected with the antisense oligonucleotides using a commercially available transfection reagent (Lipofectamine 3000, ThermoFisher Scientific), caspase activity was measured, and cytotoxicity (apoptosis-inducing activity) was inspected. The procedure is described below. The HeLa-S3 cells at the logarithmic growth phase were seeded on a 96-well plate (the plate comprises the Dulbecco's Modified Eagle Medium (DMEM) comprising 10% fetal bovine serum (FBS)) at 1.0 × 10⁴ cells/well. The antisense oligonucleotides were added to the wells to the final concentration of 200 nM 24 hours thereafter, followed by incubation for 24 hours.

Thereafter, a caspase activation reagent (Caspase-Glo3/7 Assay System, Promega) was added, and the luminescence intensity derived from Caspase-3/7 of the HeLa-S3 cells was measured. The test was performed by dual measurement (n = 2) under each condition, and the average was determined.

Figure 2 shows the results of analysis of caspase-3/7 activity. In Figure 2, the numbers on the horizontal axis correspond to the numbers (Nos.) of the antisense nucleotides shown in Tables 1 to 3. The luminescence intensities of the cells are indicated relative to the luminescence intensity of the blank sample, which is designated as 100. As a result, the antisense oligonucleotides exhibiting lower caspase-3/7 activity, compared with the cells treated with PC1 or PC2; i.e., the antisense oligonucleotides exhibiting little influence on cytotoxicity, were found. Table 4 shows antisense oligonucleotides exhibiting a small increase in the caspase-3/7 activity (apoptosis activity).

**[Table 4]**

| No. | Oligonucleotide name | Antisense (5' → 3') | Target sequence | | Caspase 3/7 activity (relative to blank %) |
|---|---|---|---|---|---|
| | | | 5' end | 3' end | |
| 1 | hCSGALNACT1-1483-AmNA(15) | 5(Am)^T(Am)^G(Am)^c^a^t^a^c^t^c^t^g^T(Am)^G(Am)^g | 1483 | 1497 | 210 |
| 5 | hCSGALNACT1-1509-AmNA(15) | T(Am)^A(Am)^G(Am)^a^g^t^a^a^a^g^c^t^A(Am)^T(Am)^c | 1509 | 1523 | 236 |
| 10 | hCSGALNACT1-1845-AmNA(15) | A(Am)^A(Am)^5(Am)^a^t^t^g^a^t^a^a^g^5(Am)^G(Am)^t | 1845 | 1859 | 176 |
| 11 | hCSGALNACT1-1846-AmNA(15) | T(Am)^A(Am)^A(Am)^c^a^t^t^g^a^t^a^a^G(Am)^5(Am)^g | 1846 | 1860 | 183 |
| 12 | hCSGALNACT1-1847-AmNA(15) | A(Am)^T(Am)^A(Am)^a^c^a^t^t^g^a^t^a^A(Am)^G(Am)^c | 1847 | 1861 | 182 |
| 15 | hCSGALNACT1-2225-AmNA(15) | G(Am)^G(Am)^A(Am)^t^t^g^t^a^c^t^g^a^5(Am)^T(Am)^g | 2225 | 2239 | 191 |
| 23 | hCSGALNACT1-3743-AmNA(15) | G(Am)^A(Am)^T(Am)^t^g^t^t^t^g^g^t^t^5(Am)^A(Am)^c | 3743 | 3757 | 156 |
| 24 | hCSGALNACT1-3744-AmNA(15) | A(Am)^G(Am)^A(Am)^t^t^g^t^t^t^g^g^t^T(Am)^5(Am)^a | 3744 | 3758 | 195 |
| 25 | hCSGALNACT1-3745-AmNA(15) | G(Am)^A(Am)^G(Am)^a^t^t^g^t^t^t^g^g^T(Am)^T(Am)^c | 3745 | 3759 | 178 |

### (Example 4) Design of scpBNA-comprising oligonucleotide etc.

Gapmers of the antisense oligonucleotides listed in Table 1 observed to suppress expression of hCSGalNAcT1 mRNA in a human glioblastoma cell line *in vitro* were designed by substituting some bases with scpBNA, changing a part of the skeleton from the phosphorothioated (PS) skeleton to the phosphodiester (PO) skeleton, and changing the sequence lengths. Thereafter, the designed gapmer antisense oligonucleotides were synthesized in the same manner as in Example 1 using an automated nucleic acid synthesizer (nS-8, GeneDesign, Inc.) on a 0.2-µmol scale or a 1.0-µmol scale. Table 2 and Table 3 show the synthesized antisense oligonucleotides.

### (Example 5) Suppression of expression of hCSGalNAcT1 mRNA in human glioblastoma cell line (YKG-1 cells) in vitro

The antisense oligonucleotides prepared in Example 4 were inspected in terms of activity of suppressing expression of hCSGalNAcT1 mRNA in the human glioblastoma cell line (YKG-1 cells) *in vitro.* As the human glioblastoma cell line, the YKG-1 cells (JCRB Cell Bank) were used. The YKG-1 cells were transfected with the antisense oligonucleotides using a commercially available transfection reagent (Lipofectamine 3000, ThermoFisher Scientific), the mRNA expression levels were measured by qRT-PCR, and the knockdown activity (suppression of mRNA expression) was inspected. The procedure is described below.

The YKG-1 cells at the logarithmic growth phase were seeded on a 24-well plate (the plate contains the Dulbecco's Modified Eagle Medium (DMEM) comprising 10% fetal bovine serum (FBS)) at 3.0 × 10⁴ cells/well. The antisense oligonucleotides were added to the wells to the final concentration of 3 nM and 30 nM 24 hours thereafter, followed by incubation for 24 hours. Thereafter, the cells were collected, and total RNA was extracted using the RNA extraction reagent (Maxwell RSC simply RNA Cells Kit, Promega) and the equipment (Maxwell RSC Instrument, Promega). cDNA was synthesized using the total RNA as a template and a reverse transcriptase (GoScript Reverse Transcriptase System, Promega). Reverse transcription was performed with a thermal cycle of 25°C for 5 minutes, 42°C for 60 minutes, 70°C for 15 minutes, and 4°C for 5 minutes. PCR amplification was performed using the synthesized cDNA as a template and a reagent for nucleic acid amplification (GoTaq qPCR Master Mix, Promega). The nucleic acid amplification reaction was performed with a thermal cycle of 95°C for 2 minutes, followed by 40 cycles of 95°C for 5 seconds and 60°C for 30 seconds. In real-time PCR, the mRNA level of the human glyceraldehyde-3-phosphate dehydrogenase (hGAPDH) of the house keeping gene was simultaneously quantified, and the mRNA level of hCSGALNACT1 relative to the mRNA level of hGAPDH was evaluated. The mRNA levels of the respective antisense oligonucleotides are shown relative to the mRNA level of the cells not treated with the oligonucleotides, which is designated as 1. The sets of primers used for PCR in the present example are the same as those used in Example 2. The test was performed by dual measurement (n = 2) under each condition, and the average was determined.

Figure 3 shows the results of analysis of suppression of mRNA expression when antisense oligonucleotides are added at 3 nM and 30 nM. Figure 4 shows the results of analysis of suppression of mRNA expression when antisense oligonucleotides are added at 3 nM and 10 nM. In all the cells treated with the antisense oligonucleotides at 10 nM or 30 nM, the mRNA levels were lower than the mRNA levels in the cells not treated with the oligonucleotides (blank cells) and the cells treated with NC.

### (Example 6) Influence of antisense oligonucleotides on scar formation (1)

Scar formation was quantified *in vitro* to examine the influence of the antisense oligonucleotides on scar formation. The present inventors developed an *in vitro* scar formation system in which fibrous scars would be formed by mixing fibroblasts and glia cells and culturing the resulting mixture in a medium comprising growth factors (TGFβ and PDGF). Scars formed as a result of restoration of tissues after injury of the spinal cord are generally considered to inhibit regeneration of nerves. Thus, recovery from the spinal cord injury can be promoted by suppressing scar formation.

U251 cells (glia cells, derived from human astrocytoma; cell registration No: IFO50288) were obtained from the National Institutes of Biomedical Innovation, Health and Nutrition, JCRB Cell Bank. HT1080 cells (fibroblasts, derived from human fibrosarcoma; cell registration No: JCRB9113) were obtained from the National Institutes of Biomedical Innovation, Health and Nutrition, JCRB Cell Bank.

The U251 cells (5 × 10⁴ cells) and the HT1080 cells (5 × 10⁴ cells) were each suspended in 500 µl of a medium (RPMI 1640 medium, Wako Pure Chemical Industries, Ltd.). The U251 cell suspension was seeded in a 4-well chamber slide (RS Glass) coated with Poly-L-lysine (SIGMA), and, 4 hours thereafter, the HT1080 cell suspension was additionally seeded. When the HT1080 cells were seeded, TGFβ (Wako Pure Chemical Industries, Ltd.) was added at 5 ng/ml. The cells were cultured in a DMEM medium supplemented with 10% FCS (SIGMA).

On the following day, the medium was exchanged with 400 µl of Opti-MEM (Gibco). At the time of medium exchange, the cells were transfected with the antisense oligonucleotides at the final concentration of 200 nM using a commercially available transfection reagent (Lipofectamine 3000, ThermoFisher Scientific). The antisense oligonucleotides Nos. 23, 30, 32, and 33 synthesized in Example 1 were used. As control cells, the cells transfected with the negative control (NC) described in Example 2 (NC was introduced into the cells) were prepared.

The cells were incubated at 37°C in a CO₂ incubator. DMEM (400 µl) supplemented with 10% FCS (SIGMA) comprising TGFβ (final concentration: 20 ng/ml) was added 6 hours later. The cells were fixed with 4% paraformaldehyde 96 hours after the administration of the antisense oligonucleotides, and images were then obtained. Based on the image data, the number of scars was determined using the image processing software (Image J, the National Institute of Health). Tests were performed at 2 different times.

Table 5 shows the number of scars when transfected with the antisense oligonucleotides. The number of scars is indicated relative to the number of scars when transfected with NC (%). All the antisense oligonucleotides Nos. 23, 30, 32, and 33 were found to decrease the number of scars.

**[Table 5]**

| No. | First | Second |
|---|---|---|
| | vs. NC (%) | vs. NC (%) |
| 23 | 41.67 | 44.19 |
| 30 | 38.89 | 32.56 |
| 32 | 8.33 | 9.30 |
| 33 | 86.11 | 67.44 |

### (Example 7) Influence of antisense oligonucleotides on scar formation (2)

In order to examine the influence of the antisense oligonucleotides Nos. 38, 39, and 41 on scar formation, evaluation was performed using the *in vitro* scar formation system in the same manner. Evaluation was performed under the same conditions as employed in Example 6, except that a 24-well plate (Thermo 142475) was used as an incubator, the U251 cells and the HT1080 cells were seeded at 3.5 × 10⁴ cells/500 µl, the HT1080 cells were seeded 5 hours after the U251 cells were seeded, and DMEM supplemented with 10% FCS comprising TGFβ was added 4 hours after transfection. The cells transfected with the positive control (PC) described in Example 2 were evaluated in the same manner. The images of the cells were obtained without fixing the cells, and the number of scars was determined.

Table 6 shows the number of scars when transfected with the antisense oligonucleotides. All the antisense oligonucleotides Nos. 38, 39, and 41 were found to exert higher effects of decreasing the number of scars than PC.

**[Table 6]**

| No. | vs. NC (%) |
|---|---|
| 38 | 38.78 |
| 39 | 40.82 |
| 41 | 1.02 |
| PC | 80.61 |

### (Example 8) Influence of antisense oligonucleotides on hindlimb motor function in spinal cord injury model mice (1)

ICR mice (wild-type) were used in the experiment. The dorsal part of the tenth vertebra (ThIO) was removed surgically from the deeply anesthetized mice. Using a device for creating spinal cord injury models (Infinite Horizon Impactor, Precision Systems and Instrumentation LLC, Lexington, NY), mice were caused to have spinal cord injury by application of a 90 k-dyne impact force.

The antisense oligonucleotide No. 30 or PBS (FUJIFILM Wako Pure Chemical Corporation) was injected with the use of a microinjector into the spinal cord tissue at the damaged site of the mice caused to have spinal cord injury simultaneously with creation of the injury. A suspension of 25 µg of the antisense oligonucleotide in PBS was administered per mouse. After the completion of administration, the wound was sutured.

The hindlimb motor functions of mice were analyzed once a week up to the 6th week. The hindlimb motor functions were analyzed by BMS open field scoring (Basso, D.M., et al., Basso Mouse Scale for locomotion detects differences in recovery after injury of the spinal cord in five common mouse strains. J Neurotrauma, 2006, 23(5): 635-59). The group of mice caused to have spinal cord injury to which PBS had been administered (the PBS group) and the group of mice caused to have spinal cord injury to which the antisense oligonucleotide No. 30 had been administered (the ASO30 group) (each group consisting of 5 mice) were subjected to the test. Mice in each group were rated by 3 to 5 evaluators. The BMS provides an overall index of the motor ability and determines the motor function recovery period and motor characteristics. The BMS is a test on a 9-point scale, where hindlimb paralysis corresponds to 0 point and the normal motor corresponds to 9 points. BMS quantitatively grades the motion of each joint of the hindlimb, weight support, the step, cooperativity of forelimbs and hindlimbs, stability of the trunk, the position of the tail in walking, and so on. A higher value indicates a higher motor function. The average and the standard deviation of each group were determined.

Figure 5 shows the results of analysis of the hindlimb motor functions of mice in each group. Figure 5A shows changes in BMS of the groups with the elapse of time. Figure 5B shows comparison of BMS of mice in each group 6 weeks after administration. An error bar in the figure indicates a standard deviation, and "**" indicates that p is less than 0.01 (p < 0.01) in the between-groups t-test. As shown in Figure 5A, satisfactory effects of motor function recovery were observed at an early stage in mice of the ASO30 group. As shown in Figure 5B, a significant difference was observed in the hindlimb motor functions between groups 6 weeks after administration.

### (Example 9) Influence of antisense oligonucleotides on hindlimb motor function in spinal cord injury model mice (2)

Self-bred C57BL/6J mice with body weight of 18 g or more were used in the experiment. The dorsal part of the tenth vertebra (ThIO) was removed surgically from the anesthetized mice. Using a device for creating spinal cord injury models (Infinite Horizon Impactor, Precision Systems and Instrumentation LLC, Lexington, NY), mice were caused to have spinal cord injury by application of a 65 k-dyne impact force, and the wound was sutured. The site of injury was incised 14 to 16 days after creation of the injury (equivalent to the subacute phase of the spinal cord injury of the mice), and the antisense oligonucleotide No. 30 or PBS (FUJIFILM Wako Pure Chemical Corporation) was injected with the use of a microinjector into the spinal cord tissue at the site of injury. A suspension of 25 µg of the antisense oligonucleotide in PBS was administered per mouse. The incised site was sutured again.

The hindlimb motor functions of mice with the spinal cord injury were analyzed once a week up to the 10th week. The hindlimb motor functions were analyzed by BMS open field scoring. The group of mice caused to have spinal cord injury to which PBS had been administered (the PBS group of 14 mice) and the group of mice caused to have spinal cord injury to which the antisense oligonucleotide No. 30 had been administered (the ASO30 group of 12 mice) were subjected to the test. Mice in each group were rated by 3 to 5 evaluators. The BMS provides an overall index of the motor ability and determines the motor function recovery period and motor characteristics. The BMS is a test on a 9-point scale, where hindlimb paralysis corresponds to 0 point and the normal motor corresponds to 9 points. BMS quantitatively grades the motion of each joint of the hindlimb, weight support, the step, cooperativity of forelimbs and hindlimbs, stability of the trunk, the position of the tail in walking, and so on. The average and the standard deviation of each group were determined.

Figure 6 shows the results of analysis of the hindlimb motor functions of mice in each group. Figure 6A shows a change in BMS of mice in each group with the elapse of time after injury of the spinal cord. The antisense nucleotide or PBS was administered 14 to 16 days after injury of the spinal cord. Figure 6B shows comparison of BMS of mice in each group 10 weeks after injury of the spinal cord. An error bar in the figure indicates a standard deviation, and "*" indicates that p is less than 0.05 (p < 0.05) in the between-groups t-test. As shown in Figure 6A, an improvement was observed in effects of motor function recovery after administration of the antisense nucleotide in mice in the ASO30 group. As shown in Figure 6B, a significant difference was observed in the hindlimb motor function between groups 10 weeks after the spinal cord injury. This indicates that the antisense oligonucleotide would exert effects of hindlimb motor function recovery on the spinal cord injury after the elapse of a given period of time after injury.

### (Example 10) Suppression of expression of CSGalNAcT1 gene in spinal cord injury marmoset models

The fifth cervical vertebra of common marmosets was injured to prepare spinal cord injury models. The artificial cerebral spinal fluid supplemented with the antisense oligonucleotide No. 30 was continuously administered using an osmotic pump through a catheter retained in the subarachnoid cavity of the spinal cord. The antisense oligonucleotide was administered at a dose of 0 µg/day (negative control) and 100 µg/day per mouse. Two marmosets (No. 1 and No. 2) were subjected to administration of negative controls, and a marmoset (No. 3) was subjected to administration at 100 µg/day. Two weeks after the initiation of administration, the spinal cord was extracted from each common marmoset, and the CSGalNAcT1 mRNA level (expression level) in the spinal cord was measured by quantitative PCR. The plasma samples obtained simultaneously with spinal cord sampling were used to measure the blood biochemical indices of marmosets.

Figure 7 shows the CSGalNAcT1 mRNA level in the spinal cord of each marmoset. In the figure, the expression level is indicated relative to the expression of a negative control mouse (No. 1), which is designated as 1. The CSGalNAcT1 gene expression level was lowered in the spinal cord of a marmoset to which the antisense oligonucleotide had been administered. Concerning the measured blood biochemical indices, no significant difference was observed between the negative control and the marmoset to which the antisense oligonucleotide had been administered. This indicates that administration of the antisense nucleotide can effectively suppress expression of the CSGalNacT1 gene without significantly influencing other blood biochemical indices in marmosets, the Primates.

All publications, patents, and patent applications cited herein are incorporated herein by reference in their entirety.

## Claims

1. An antisense oligonucleotide that suppresses expression of the chondroitin sulfate N-acetylgalactosaminyl-transferase-1 (CSGalNAc-T1) gene and has the following characteristics:
(i) an antisense oligonucleotide is 13- to 25-mer in length;
(ii) an antisense oligonucleotide comprises a gap region, a 3'-wing region bound to the 3' end of the gap region, and a 5'-wing region bound to the 5' end of the gap region;
(iii) nucleotides in the 3'-wing region and in the 5'-wing region include at least one bridged nucleic acid and the bridged nucleic acid is scpBNA or AmNA; and
(iv) an antisense oligonucleotide comprises a nucleotide sequence selected from the group consisting of SEQ ID NOs: 2 to 31 or a sequence derived therefrom by substitution, deletion, or insertion of 1 or 2 nucleic acid bases.

2. The antisense oligonucleotide according to claim 1, wherein the nucleotides in the 3'-wing region and in the 5'-wing region include at least one scpBNA.

3. The antisense oligonucleotide according to claim 1, wherein at least one internucleotide bond is a phosphorothioate bond.

4. The antisense oligonucleotide according to claim 1, wherein at least one internucleotide bond in the 3'-wing region and in the 5'-wing region is a phosphodiester bond.

5. The antisense oligonucleotide according to claim 4, wherein the proportion of phosphodiester (PO) bonds relative to the total of phosphorothioate (PS) bonds and PO bonds (PO/(PS+PO)) of the internucleotide bonds in the 3'-wing region and in the 5'-wing region is less than 0.5.

6. The antisense oligonucleotide according to claim 1, wherein the gap region is 7- to 17-mer in length, the 3'-wing region is 2- to 6-mer in length, and the 5'-wing region is 2- to 6-mer in length.

7. The antisense oligonucleotide according to claim 1, which has a nucleotide sequence selected from the group consisting of SEQ ID NOs: 24, 26, 27, 29, and 31 or a nucleotide sequence derived therefrom by substitution, deletion, or insertion of 1 or 2 nucleic acid bases.

8. A pharmaceutical composition comprising the antisense oligonucleotide according to any one of claims 1 to 7 used for treating a disease or condition related to an increase in chondroitin sulfate.

9. The pharmaceutical composition according to claim 8, wherein the disease or condition related to an increase in chondroitin sulfate is a spinal cord injury.

10. The pharmaceutical composition according to claim 9, which is administered at the acute phase or the subacute phase of a spinal cord injury.

11. The pharmaceutical composition according to claim 10, which is administered at the subacute phase of a spinal cord injury.
